# EUROPÄISCHE PATENTANMELDUNG

(11) **EP 0 722 937 A1**
(43) Veröffentlichungstag der Anmeldung: **24.07.1996**
(21) Anmeldenummer: 96100154.2
(22) Anmeldetag: 08.01.1996
(51) Int. Cl.: C07D 239/91, C07D 405/04, A61K 31/505

(54) **2,8-Disubstituierte Chinazolinone**

(30) Priorität: 19.01.1995 DE 19501481
(71) Anmelder: BAYER AG, 51368 Leverkusen (DE)
(72) Erfinder: Heiker, Fred R., Dr., D-42113 Wuppertal (DE); Niewöhner, Ulrich, Dr., D-42929 Wermelskirchen (DE); Hartwig, Wolfgang, Dr., Stamford, Connecticut 06903 (US); Schütz, Helmuth, Dr., D-42115 Wuppertal (DE); Bischoff, Erwin, Dr., D-42115 Wuppertal (DE); Perzborn, Elisabeth, Dr., D-42327 Wuppertal (DE); Schramm, Matthias, Dr., D-51375 Leverkusen (DE)

(57) **Zusammenfassung**

2,8-Disubstituierte Chinazolinone werden hergestellt, indem man zunächst durch übliche Reaktionen das Chinazolinongrundgerüst kondensiert und anschließend den gewünschten Substituenten in 8-Position einführt. Die Verbindungen eignen sich als Wirkstoffe in Arzneimitteln, insbesondere zur Behandlung von Entzündungen, thromboembolischen Erkrankungen sowie Herz-/Kreislauferkrankungen.

## Beschreibung

Die vorliegende Erfindung betrifft 2,8-disubstituierte Chinazolinone, ein Verfahren zu ihrer Herstellung und ihre Verwendung in Arzneimitteln, insbesondere zur Behandlung von Entzündungen, thromboembolische, Herz- und Kreislauferkrankungen und Erkrankungen des Urogenitalsystems.

Aus der Publikation PCT WO 93/12095 sind Chinazolinone mit einer selektiven cGMP PDE inhibitorischen Wirkung bekannt.

Phosphodiesterasen (PDE's) spielen eine wesentliche Rolle in der Regulation des intrazellulären cGMP und cAMP-Spiegels. Von den bisher beschriebenen Phosphodiesterase-Isoenzymgruppen PDE I bis PDE V [Nomenklatur nach Beavo and Reifsnyder (vgl. Beavo, J.A. and Reifsnyder, D.H.: Trends in Pharmacol. Sci. 11, 150-155 (1990))] sind die Ca-Calmodulin aktivierte PDE I, die cGMP stimulierbare PDE II und die cGMP spezifische PDE V im wesentlichen für den Metabolismus von cGMP verantwortlich. Aufgrund der unterschliedlichen Verteilung dieser cGMP metabolisierenden PDE's im Gewebe sollten selektive Inhibitoren je nach Gewebsverteilung des entsprechenden Isoenzyms die c-GMP-Spiegel im entsprechenden Gewebe anheben. Dieses kann zu einer spezifischen, antiaggregatorischen, antispastischen, gefäßdilatierenden, antiarrhythmischen und/oder antiinflammatorischen Wirkung führen.

Die vorliegende Erfindung betrifft nun 2,8-disubstituierte Chinazolinone- der allgemeinen Formel (I), 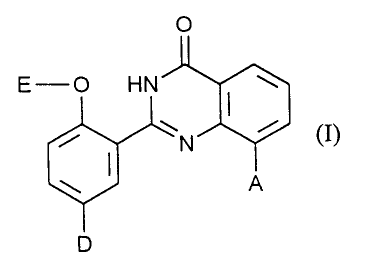
in welcher
- A: für Oxiranyl steht, das gegebenenfalls durch geradkettiges oder verzweigtes Alkyl mit bis zu 8 Kohlenstoffatomen substituiert ist, das seinerseits durch Phenyl substituiert sein kann, oder
für einen Rest der Formel 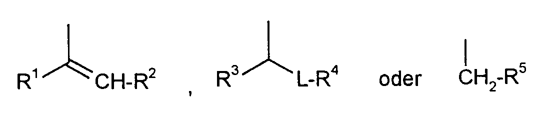 steht,
worin
- R¹: Wasserstoff oder geradkettiges oder verzweigtes Alkyl mit bis zu 6 Kohlenstoffatomen bedeutet,
- R²: geradkettiges oder verzweigtes Alkyl mit bis zu 8 Kohlenstoffatomen bedeutet, das gegebenenfalls durch Phenyl substituiert ist,
- R³: geradkettiges oder verzweigtes Alkyl mit bis zu 5 Kohlenstoffatomen oder eine Gruppe der Formel -OR⁶ bedeutet,
worin
- R⁶: Wasserstoff, eine Hydroxyschutzgruppe oder geradkettiges oder verzweigtes Alkyl mit bis zu 5 Kohlenstoffatomen bedeutet,
- R⁴: geradkettiges oder verzweigtes Alkyl mit 2 bis 10 Kohlenstoffatomen bedeutet, das gegebenenfalls durch Phenyl substituiert ist,
- L: einen Rest der Formel -CO-, -CH(OH), -CH₂, -CH(N₃) oder -CH(OSO₂R⁷) bedeutet,
worin
- R⁷: geradkettiges oder verzweigtes Alkyl mit bis zu 4 Kohlenstoffatomen oder Phenyl bedeutet,
- R⁵: geradkettiges oder verzweigtes Alkyl mit 3 bis 8 Kohlenstoffatomen bedeutet, das gegebenenfalls durch Phenyl substituiert ist, oder Benzyl oder 2-Phenylethyl bedeutet,
- D: für Wasserstoff oder für eine Gruppe der Formel -SO₂-NR⁸R⁹ steht,
worin
- R⁸ und R⁹: gleich oder verschieden sind und
Wasserstoff, Phenyl oder geradkettiges oder verzweigtes Alkyl mit bis zu 6 Kohlenstoffatomen bedeuten, das gegebenenfalls durch Hydroxy substituiert ist,
oder gemeinsam mit dem Stickstoffatom einen 5- bis 6-gliedrigen gesättigten Heterocyclus mit bis zu 2 weiteren Heteroatomen aus der Reihe S, N und/oder O bilden, der gegebenenfalls, auch über eine freie N-Funktion, durch geradkettiges oder verzweigtes Alkyl mit bis zu 6 Kohlenstoffatomen substituiert ist, das seinerseits durch Hydroxy substituiert sein kann,
- E: für geradkettiges oder verzweigtes Alkyl mit bis zu 8 Kohlenstoffatomen steht
und deren Tautomere und Salze.

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen der allgemeinen Formel (I) können in verschiedenen stereochemischen Formen auftreten, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Ein über das Stickstoffatom gebundener, 5- bis 6-gliedriger gesättigter Heterocyclus, der außerdem als Heteroatome bis zu 2 Sauerstoff-, Schwefel- und/oder Stickstoffatome enthalten kann, steht im allgemeinen für Piperidyl, Morpholinyl oder Piperazinyl. Besonders bevorzugt ist Morpholinyl.

Hydroxyschutzgruppe im Rahmen der oben angegebenen Definition steht im allgemeinen für eine Schutzgruppe aus der Reihe: Trimethylsilyl, Triethylsilyl, Triisopropylsilyl, tert.Butyl-dimethylsilyl, Triphenylsilyl oder Benzyl. Bevorzugt sind Trimethylsilyl, tert.Butyl-dimethylsilyl oder Benzyl.

Bevorzugt sind Verbindungen der allgemeinen Formel (I),
in welcher
- A: für Oxiranyl steht, das gegebenenfalls durch geradkettiges oder verzweigtes Alkyl mit bis zu 7 Kohlenstoffatomen substituiert ist, das seinerseits durch Phenyl substituiert sein kann, oder
für einen Rest der Formel 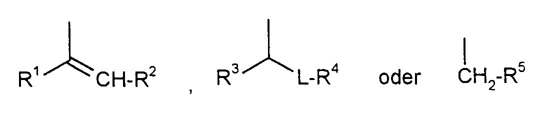 steht,
worin
- R¹: Wasserstoff oder geradkettiges oder verzweigtes Alkyl mit bis zu 5 Kohlenstoffatomen bedeutet,
- R²: geradkettiges oder verzweigtes Alkyl mit bis zu 6 Kohlenstoffatomen bedeutet, das gegebenenfalls durch Phenyl substituiert ist,
- R³: geradkettiges oder verzweigtes Alkyl mit bis zu 4 Kohlenstoffatomen oder eine Gruppe der Formel -OR⁶ bedeutet,
worin
- R⁶: Wasserstoff, Benzyl, Acetyl oder geradkettiges oder verzweigtes Alkyl mit bis zu 4 Kohlenstoffatomen bedeutet,
- R⁴: geradkettiges oder verzweigtes Alkyl mit 2 bis 8 Kohlenstoffatomen bedeutet, das gegebenenfalls durch Phenyl substituiert ist,
- L: einen Rest der Formel -CO-, -CH(OH), -CH₂, -CH(N₃) oder -CH(OSO₂R⁷) bedeutet,
worin
- R⁷: geradkettiges oder verzweigtes Alkyl mit bis zu 3 Kohlenstoffatomen oder Phenyl bedeutet,
- R⁵: geradkettiges oder verzweigtes Alkyl mit 3 bis 7 Kohlenstoffatomen bedeutet, das gegebenenfalls durch Phenyl substituiert ist, oder Benzyl oder 2-Phenylethyl bedeutet,
- D: für Wasserstoff oder für eine Gruppe der Formel -SO₂-NR⁸R⁹ steht,
worin
- R⁸ und R⁹: gleich oder verschieden sind und
Wasserstoff, Phenyl oder geradkettiges oder verzweigtes Alkyl mit bis zu 5 Kohlenstoffatomen bedeuten, das gegebenenfalls durch Hydroxy substituiert ist,
oder gemeinsam mit dem Stickstoffatom einen Morpholinyl-, Piperidinyl- oder Piperazinylring bilden, der gegebenenfalls, auch über eine freie N-Funktion, durch geradkettiges oder verzweigtes Alkyl mit bis zu 4 Kohlenstoffatomen substituiert ist, das seinerseits durch Hydroxy substituiert sein kann,
- E: für geradkettiges oder verzweigtes Alkyl mit bis zu 6 Kohlenstoffatomen steht
und deren Tautomere und Salze.

Besonders bevorzugt sind Verbindungen der allgemeinen Formel (I),
in welcher
- A: für Oxiranyl steht, das gegebenenfalls durch geradkettiges oder verzweigtes Alkyl mit bis zu 6 Kohlenstoffatomen substituiert ist, das seinerseits durch Phenyl substituiert sein kann, oder
für einen Rest der Formel 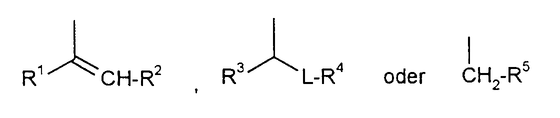 steht,
worin
- R¹: Wasserstoff oder geradkettiges oder verzweigtes Alkyl mit bis zu 3 Kohlenstoffatomen bedeutet,
- R²: geradkettiges oder verzweigtes Alkyl mit bis zu 6 Kohlenstoffatomen bedeutet, das gegebenenfalls durch Phenyl substituiert ist,
- R³: geradkettiges oder verzweigtes Alkyl mit bis zu 4 Kohlenstoffatomen oder eine Gruppe der Formel -OR⁶ bedeutet,
worin
- R⁶: Wasserstoff, Benzyl, Acetyl oder geradkettiges oder verzweigtes Alkyl mit bis zu 3 Kohlenstoffatomen bedeutet,
- R⁴: geradkettiges oder verzweigtes Alkyl mit 2 bis 7 Kohlenstoffatomen bedeutet, das gegebenenfalls durch Phenyl substituiert ist,
- L: einen Rest der Formel -CO-, -CH(OH), -CH₂, -CH(N₃) oder -CH(OSO₂R⁷) bedeutet,
worin
- R⁷: geradkettiges oder verzweigtes Alkyl mit bis zu 3 Kohlenstoffatomen oder Phenyl bedeutet,
- R⁵: geradkettiges oder verzweigtes Alkyl mit 3 bis 6 Kohlenstoffatomen bedeutet, das gegebenenfalls durch Phenyl substituiert ist, oder Benzyl oder 2-Phenylethyl bedeutet,
- D: für Wasserstoff oder für eine Gruppe der Formel -SO₂-NR⁸R⁹ steht,
worin
- R⁸ und R⁹: gleich oder verschieden sind und Wasserstoff, Phenyl oder geradkettiges oder verzweigtes Alkyl mit bis zu 3 Kohlenstoffatomen bedeuten, oder gemeinsam mit dem Stickstoffatom einen Morpholinyl- oder Piperidinylring bilden,
- E: für geradkettiges oder verzweigtes Alkyl mit bis zu 4 Kohlenstoffatomen steht
und deren Tautomere und Salze.

Außerdem wurde ein Verfahren zur Herstellung der erfindungsgemäßen Verbindungen der allgemeinen Formel (I) gefunden, dadurch gekennzeichnet, daß man zunächst Verbindungen der allgemeinen Formel (II) 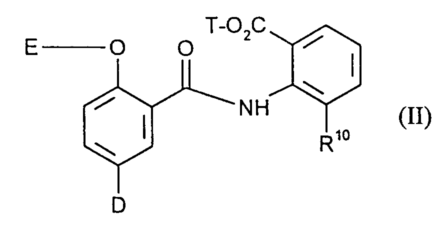
in welcher
D und die oben angegebene Bedeutung haben,
T für C₁-C₄-Alkyl steht
und
R¹⁰ für Halogen, vorzugsweise für Brom oder Jod steht mit Formamid zu den Verbindungen der allgemeinen Formel (III) 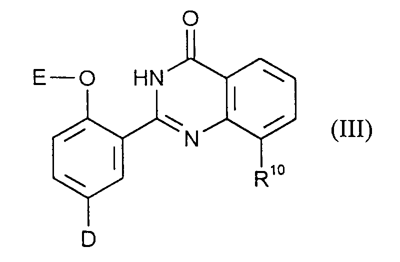
in welcher
D, E und R¹⁰ die oben angegebene Bedeutung haben,
cyclisiert,
und in einem letzten Schritt mit Verbindungen der allgemeinen Formel (IV)

R¹-CH=CH-R² (IV)

in welcher
R¹ und R² die oben angegebene Bedeutung haben,
in inerten Lösemitteln, in Anwesenheit einer Base und im System Tri-o-tolylphosphin/Palladium-II-acetat in die Verbindungen der allgemeinen Formel (Ia) 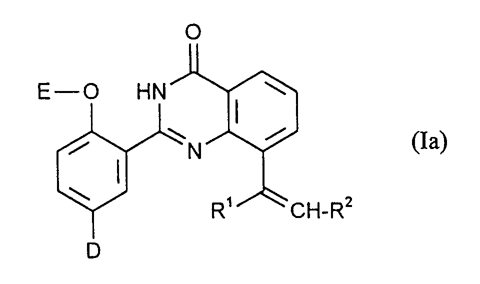
in welcher
D, E, R¹ und R² die oben angegebene Bedeutung haben,
überführt,
und gegebenenfalls die Doppelbindung hydriert,
oder im Fall A = substituiertes Oxiranyl,
gegebenenfalls die Doppelbindung nach üblichen Methoden mit einem Oxidationsmittel in inerten Lösemitteln zu den entsprechenden Epoxyverbindungen oxidiert und diese durch Ringöffnungsreaktionen in die entsprechenden Hydroxyverbindungen überführt,
und ausgehend von den Hydroxyverbindungen, gegebenenfalls nach Aktivierung, nucleophile Substitutionsreaktionen durchführt,
oder die Hydroxyverbindungen zu den Oxoverbindungen oxidiert.

Das erfindungsgemäße Verfahren kann durch folgendes Formelschema beispielhaft erläutert werden: 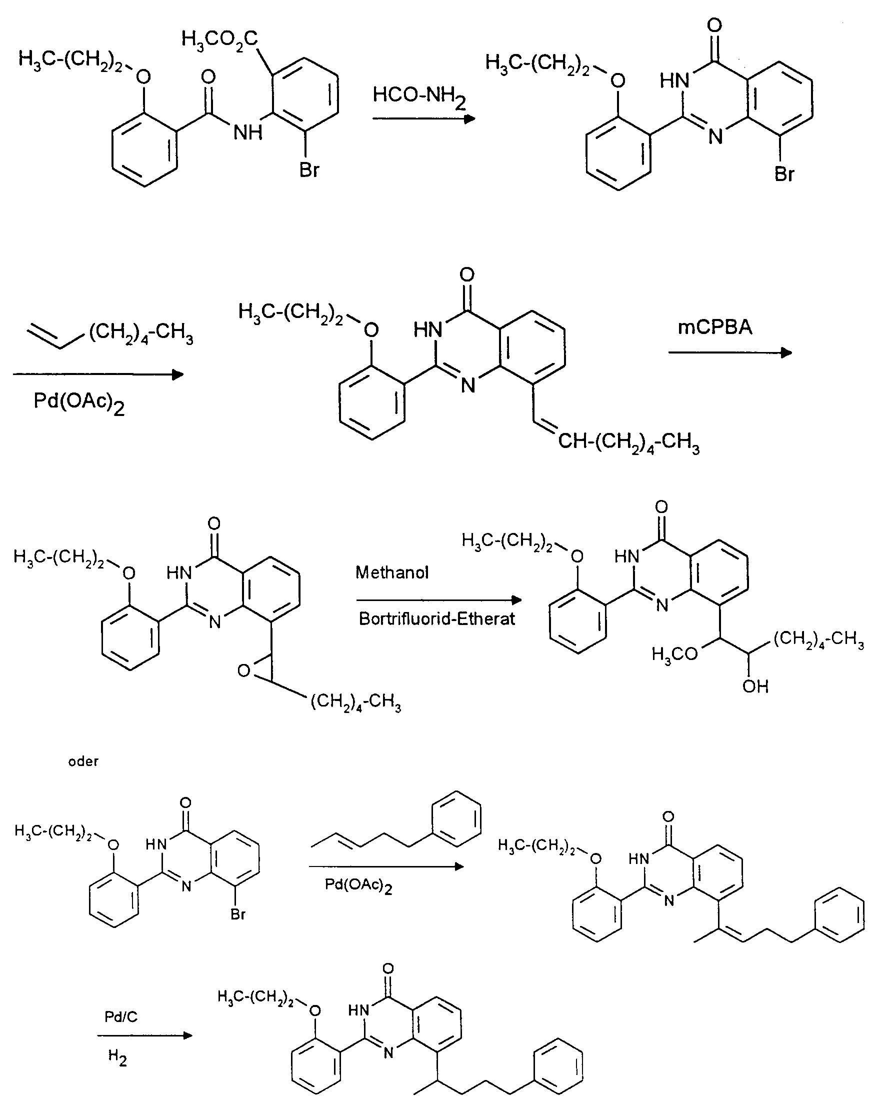
Für das Verfahren eignen sich inerte organische Lösemittel, die sich unter den Reaktionsbedingungen nicht verändern. Hierzu gehören bevorzugt Ether wie beispielsweise Diethylether, Dioxan, Tetrahydrofuran, Glykolmono- oder dimethylether, Halogenkohlenwasserstoffe wie Di-, Tri- oder Tetrachlormethan, Dichlorethylen, Trichlorethylen, Essigester, Toluol, Acetonitril, Dimethylformamid, Hexamethylphosphorsäuretriamid und Aceton. Selbstverständlich ist es möglich, Gemische der Lösemittel einzusetzen. Besonders bevorzugt sind Dichlormethan und Dimethylformamid.

Die Reaktionstemperatur kann im allgemeinen in einem größeren Bereich variiert werden. Im allgemeinen arbeitet man in einem Bereich von -20°C bis 200°C, bevorzugt von 0°C bis 25°C.

Die Cyclisierung wird im allgemeinen in einem Temperaturbereich von +50°C bis 200°C, bevorzugt von +160°C bis +180°C durchgeführt.

Die Herstellung der Verbindungen der allgemeinen Formel (Ia) erfolgt im allgemeinen in einem der oben aufgeführten Lösemittel, vorzugsweise Dimethylformamid, und in Anwesenheit einer Base.

Als Basen können im allgemeinen anorganische oder organische Basen eingesetzt werden. Hierzu gehören vorzugsweise Alkalicarbonate wie Natriumcarbonat, Kaliumcarbonat oder Caesiumcarbonat, oder Alkali- oder Erdalkalialkoholate oder -amide wie Natrium- oder Kaliummethanolat, Natrium- oder Kaliumethanolat, Kalium-tert.butylat oder Kaliumamid, oder organische Amine (Trialkyl(C₁-C₆)amine) wie Triethylamin oder Tributylamin. Besonders bevorzugt ist Tributylamin.

Im allgemeinen setzt man die Base in einer Menge von 0,05 Mol bis 10 Mol, bevorzugt von 1 Mol bis 2 Mol bezogen auf 1 Mol der Verbindung der Formel (III) ein.

Das erfindungsgemäße Verfahren wird im allgemeinen in einem Temperaturbereich von 0°C bis +180°C, bevorzugt von +30°C bis +150°C durchgeführt.

Die erfindungsgemäßen Verfahrenschritte werden im allgemeinen bei Normaldruck durchgeführt. Es ist aber auch möglich, bei Überdruck oder bei Unterdruck zu arbeiten (z.B. in einem Bereich von 0,5 bis 5 bar).

Die Epoxidierung erfolgt in einem der oben aufgeführten Lösemittel, vorzugsweise trockenem Trichlormethan, in Anwesenheit eines Oxidationsmittels, wie beispielsweise m-Chlorbenzoesäure oder H₂O₂. Bevorzugt ist m-Chlorperbenzoesäure.

Die Epoxidierung wird im allgemeinen in einem Temperaturbereich von -20°C bis +50°C, bevorzugt von 0°C bis +30°C durchgeführt.

Die Hydrierung erfolgt im allgemeinen in einem der oben aufgeführten Alkohole, vorzugsweise Methanol.

Als Katalysator eignen sich im allgemeinen Palladiumverbindungen. Bevorzugt ist Pd/C.

Der Katalysator wird in einer Menge von 0,01 Mol bis 0,4 Mol, bevorzugt von 0,05 Mol bis 0,2 Mol bezogen auf 1 Mol des entsprechenden Alkohols eingesetzt.

Die Hydrierung wird im allgemeinen in einem Temperaturbereich von -20°C bis +50°C, bevorzugt von 0°C bis +30°C durchgeführt.

Die Hydrierung wird im allgemeinen bei Normaldruck durchgeführt. Es ist aber auch möglich, bei Überdruck oder bei Unterdruck zu arbeiten (z.B. in einem Bereich von 0,5 bis 5 bar).

Die Epoxidöffnungen erfolgen nach dem in der Literatur beschriebenen Methoden [vgl.Takano et al., Heterocycles 29, (1989), 249] und ebenfalls in einem der oben aufgeführten Alkohole, vorzugsweise Methanol in Anwesenheit von Bortrifluorid-Etherat.

Die Umsetzung mit Alkylsulfonsäurechloriden erfolgt, ausgehend von den entsprechenden freien Hydroxyverbindungen, in einem der oben aufgeführten Lösemittel und einer der Basen, vorzugsweise mit Dichlormethan und Triethylamin in einem Temperaturbereich von -20C bis +20°C, vorzugsweise 0°C und Normaldruck.

Die Einführung des Azidrestes erfolgt im allgemeinen durch Umsetzung der entsprechenden Alkylsulfonyloxy substituierten Verbindungen mit Natriumazid in einem der oben aufgeführten Lösemittel, vorzugsweise Dimethylformamid, in einem Temperaturbereich von 50°C bis +120°C, vorzugsweise 100°C und Normaldruck.

Die Ketone werden ausgehend von den entsprechenden Hydroxyverbindungen nach bekannten Methoden (Swern-Oxidation) hergestellt.

Die enantiomerenreinen Verbindungen sind nach üblichen Methoden, beispielsweise durch Chromatographie der racemischen Verbindungen der allgemeinen Formel (I) auf chiralen Phasen zugänglich.

Die Verbindungen der allgemeinen Formel (II) sind teilweise bekannt oder neu und können dann hergestellt werden, indem man Verbindungen der allgemeinen Formel (V) 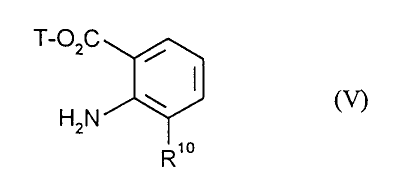
in welcher
R¹⁰ und T die oben angegebene Bedeutung haben
durch Umsetzung mit 2-n-Alkoxybenzoesäurechloriden der Formel (VI) 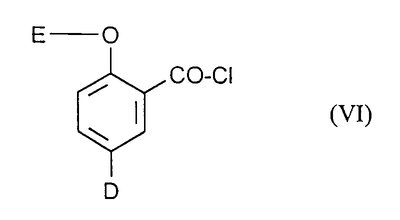
in welcher
D und E die oben angegebene Bedeutung haben,
in inerten Lösemitteln und in Anwesenheit einer Base umsetzt.

Als Lösemittel eignen sich die oben aufgeführten Lösemitteln, wobei Dichlormethan bevorzugt ist.

Als Basen eignen sich cyclische Amine, wie beispielsweise Piperidin, Pyridin, Pyrimidin, Dimethylaminopyridin oder C₁-C₄-Alkylamine, wie beispielsweise Triethylamin. Bevorzugt sind Triethylamin und Pyridin.

Die Base wird im allgemeinen in einer Menge von 0,5 Mol bis 2 Mol, bevorzugt von 1 Mol bis 1,2 Mol jeweils bezogen auf 1 Mol der Verbindungen der allgemeinen Formel (V) eingesetzt.

Die Reaktionstemperatur kann im allgemeinen in einem größeren Bereich variiert werden. Im allgemeinen arbeitet man in einem Bereich von -20°C bis 200°C, bevorzugt von 0°C bis 25°C.

Die Verbindungen der allgemeinen Formeln (V) (z.B. J. Heterocyclic. Chem., 26(5), 1989, 1405-1413) und (VI) (z.B. EP-0 526 004 A1) sind an sich bekannt.

Die Verbindungen der allgemeinen Formel (III) sind neu und können wie oben beschrieben hergestellt werden.

Die Verbindungen der allgemeinen Formel (IV) sind bekannt.

Die Verbindungen der allgemeinen Formel (Ia) sind neu und können wie oben beschrieben hergestellt werden.

Die erfindungsgemäßen Verbindungen der allgemeinen Formel (I) und (Ia) zeigen ein nicht vorhersehbares, wertvolles pharmakologisches Wirkspektrum.

Sie inhibieren entweder eine oder mehrere der c-GMP metabolisierenden Phosphodiesterasen (PDE I, PDE II und PDE V). Dies führt zu einem differenzierten Anstieg von c-GMP. Eine Erhöhung des c-GMP-Spiegels kann zu einer antithrombotischen, vasodilatorischen, antiarrhythmischen und/oder antientzündlichen Wirkung führen. Die Selektivität wird von der Verteilung der Isoenzyme im Gewebe mitbestimmt.

Außerdem verstärken die erfindungsgemäßen Verbindungen die Wirkung von Substanzen, wie beispielsweise EDRF (Endothelium derived relaxing factor) und ANP (atrial natriuretic peptide), die den cGMP-Spiegel steigern.

Sie können daher in Arzneimitteln zur Behandlung von entzündlichen Erkrankungen wie beispielsweise Asthma, entzündlichen Dermatosen, zur Behandlung des Bluthochdrucks, stabiler und instabiler Angina, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, Angina pectoris, periphere Durchblutungsstörungen, Verhinderung von Restenosen wie nach Thrombolysetherapien, percutan transluminalen Angioplastien (PTA) und Bypass, percutan transluminalen Koronarangioplastien (PTCA), Bypass, septischem Schock und Krankheiten des Urogenitalsystems wie beispielsweise Prostatahypertrophy, Impotenz und Inkontinenz eingesetzt werden.

### Aktivität der Phosphordiesterasen (PDE's)

Die c-GMP stimulierbare PDE II, die c-GMP hemmbare PDE III und die cAMP spezifische PDE IV wurden entweder aus Schweine- oder Rinderherzmyokard isoliert. Die Ca-Cal-modulinstimulierbare PDE I wurde aus Schweineaorta oder Schweinegehirn isoliert. Die c-GMP spezifische PDE V wurde aus Schweinedünndarm, Schweineaorta und/oder humanen Blutplättchen gewonnen. Die Reinigung erfolgte durch Anionenaustauschchromatographie an MonoQ^{R} Pharmacia im wesentlichen nach der Methode von M. Hoey and Miles D. Houslay, Biochemical Pharmacology, Vol. 40, 193-202 (1990).

Die Bestimmung der Enzymaktivität erfolgt in einem Testansatz von 100 µl in 20 mM Tris/HCl-Puffer pH 7,5 der 5 mM MgCl₂, 0,1 mg/ml Rinderserumalbumin und entweder 800 Bq ³HcAMP oder ³HcGMP enthält. Die Endkonzentration der entsprechenden Nucleotide ist 10⁻⁶ mol/l. Die Reaktion wird durch Zugabe des Enzyms gestartet, die Enzymmenge ist so bemessen, daß während der Inkubationszeit von 30 min ca 50% des Substrates umgesetzt werden. Um die cGMP stimulierbare PDE II zu testen, wird als Substrat ³HcAMP verwendet und dem Ansatz 10⁻⁶ Mol/l nicht markiertes cGMP zugesetzt. Um die Ca-Calmodulinabhängige PDE I zu testen, werden dem Reaktionsansatz noch CaCl₂ 1 µM und Calmodulin 0,1 µM zugesetzt. Die Reaktion wird durch Zugabe von 100 µl Acetonitril, das 1 mM cAMP und 1 mM AMP enthält, gestoppt. 100 µl des Reaktionsansatzes werden auf der HPLC getrennt und die Spaltprodukte "Online" mit einem Durchflußscintillationszähler quantitativ bestimmt. Es wird die Substanzkonzentration gemessen, bei der die Reaktionsgeschwindigkeit um 50% vermindert ist.

### Inhibition der Phosphodiesterasen in vitro

| Bsp.-Nr. | PDE I IC₅₀ [nM] | PDE II IC₅₀ [nM] | PDE V IC₅₀ [nM] |
|---|---|---|---|
| 14 | 5 | 5 | 3 |
| 15 | 3 | 5 | |
| 16 | | 5 | 5 |
| 20 | 10 | 2 | 5 |
| 29 | | 0,5 | 1 |

Die Verbindungen wurden auf antihypertensive Aktivität am narkotisierten Schwein untersucht.

Die antihypertensive Aktivität wurde nach intravenöser Applikation an SHR-Ratten gemessen.

Zur Bestimmung der cyclischen Nucleotide wurden Herz- und Aortengewebe entnommen und unmittelbar tiefgefroren. Die Proben wurden unter flüssigen N₂ pulverisiert, mit 70% Ethanol extrahiert und der Gehalt am cGMP und cAMP mit kommerziellen Radioimmunoassays (Amersham) bestimmt.

Die erektionsauslösende Wirkung wurde am narkotisierten Kaninchen gemessen. (C.G. Stief et al. World Journal Urology 1990, S. 233-236).

Die Substanzen wurden in Dosierungen 0,1 bis 10 mg/kg entweder direkt in den Corpus cavernosum, intraduodenal, rektal, oral, transdermal oder intravenös appliziert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, parenteral, transdermal, perlingual oder intravenös.

Im allgemeinen hat es sich als vorteilhaft erwiesen, bei intravenöser Applikation Mengen von etwa 0,01 bis 10 mg/kg, vorzugsweise etwa 0,1 bis 10 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindungen

### Beispiel I

### 2-(2-n-Propoxybenzamido)-3-jod-benzoesäuremethylester

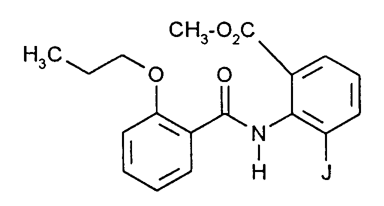

27,9 g (0,1 Mol) 2-Amino-3-jod-benzoesäuremethylester und 15,4 ml (0,11 Mol) Triethylamin wurden in 170 ml abs. CH₂Cl₂ gelöst. Bei 0°C wurde eine Lösung von 20 g (0,1 Mol) 2-n-Propoxybenzoesäurechlorid in 80 ml abs. CH₂Cl₂ zugetopft. Man rührt über Nacht bei 20°C, filtriert den Niederschlag ab und schüttelt mit 100 ml 1 n HCl, 100 ml 1 n NaOH und 100 ml gesättigter NaCl-Lösung aus. Die organische Phase wird über Na₂SO₄ getrocknet, im Vakuum eingedampft und der Rückstand durch Chromatographie über Kieselgel (Eluens: Toluol/Essigsäureethylester 95:5)) gereinigt.
Ausbeute: 36 g (81,4%)
R_{f} = 0,25 (Toluol/Essigsäureethylester 10:1)

### Beispiel II

### 2-(2-n-Propoxybenzamido)-3-brom-benzoesäuremethylester

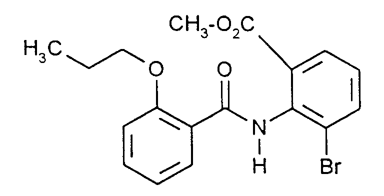

Analog der Vorschrift für Beispiel I wurde die Titelverbindung ausgehend von 2-Amino-3-brom-benzoesäuremethylester hergestellt.
Ausbeute: 60,4%
R_{f} = 0,19 (Toluol/Essigsäureethylester 5:1)

### Beispiel III

### 2-(2-n-Propoxyphenyl)-8-jod-chinazolin-4-(3H)-on

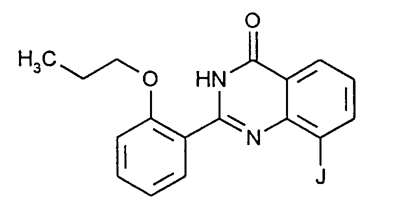

19,4 g (44,17 mMol) der Verbindung aus Beispiel I wurden 10 h bei 180°C in 216 ml Formamid gerührt. Nach dem Abkühlen wurden 500ml Wasser zugesetzt und 4 mal mit je 300 ml CH₂Cl₂ extrahiert. Die vereingten organischen Phasen wurden über MgSO₄ getrocknet, das Lösemittel im Vakuum eingedampft und der Rückstand in einem Gemisch aus 100 ml Diethylether und 50 ml Petrolether verrührt. Das Produkt wurde abgesaugt (17,8 g) und aus 250 ml abs. Ethanol umkristallisiert.
Ausbeute: 14,56 g (81,2%)
Fp.: 174°C

### Beispiel IV

2-(2-n-Propoxyphenyl)-8-brom-chinazolin-4-(3H)-on 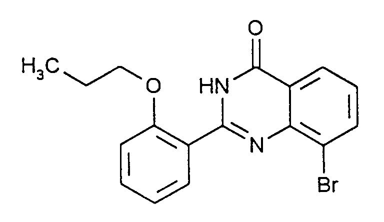
Analog der Vorschrift für Beispiel III wurde die Titelverbindung ausgehend von der Verbindung aus Beispiel II hergestellt.
Ausbeute: 60%
R_{f} = 0,7 (Toluol/Essigsäureethylester 10:1)

### Herstellungsbeispiele

### Beispiel 1

### 2-(2-n-Propoxyphenyl)-8-(1-hepten-1-yl)-chinazolin-4(3H)-on

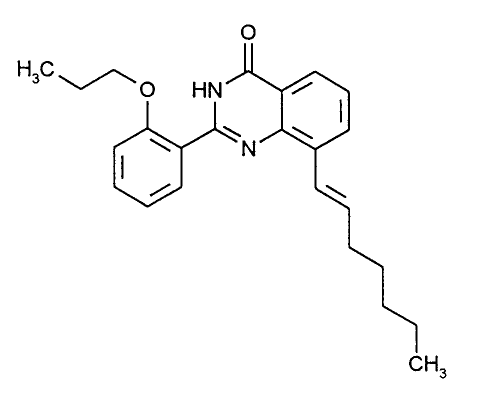

5 g (12,31 mMol) der Verbindung aus Beispiel III, 3,7 ml (15,4 mMol) Tributylamin, 6,6 ml (46,2 mMol) 1-Hepten, 375 mg Tri-o-tolylphosphin (1,23 mMol) und 138 mg Palladium(II)acetat (0,6 mMol) wurden in 50 ml trockenem DMF 2,5 h bei 100°C gerührt. Es wurde auf Raumtemperatur abgekühlt und nach Zugabe von 50 ml Essigsäureethylester 3 mal mit je 50 ml H₂O gewaschen. Nach Trocknen über MgSO₄ wurde die organische Phase im Vakuum eingedampft und der Rückstand über Kieselgel mit Toluol/Essigsäureethylester 95:5 als Eluens chromatographiert. Die das Produkt enthaltenen Fraktionen wurden vereinigt und das Lösemittel im Vakuum eingedampft. Der zunächst ölige Rückstand wurde durch Verrühren mit 35 ml Petrolether kristallisiert.
Ausbeute: 2,2 g (47,5%)
Fp.: 94°C

### Beispiel 2

### 2-(2-n-Propoxyphenyl)-8-(3-phenyl-1-propen-1-yl)-chinazolin-4(3H)-on

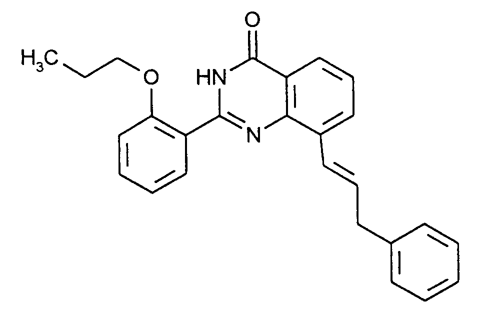

In Analogie zur Vorschrift des Beispiels 1 wurde die Titelverbindung ausgehend von der Verbindung des Beispiels III und 3-Phenyl-1-propen erhalten.
Ausbeute: 63,9%
Fp. 123-126°C (aus Diethylether)

### Beispiel 3

### 2-(2-n-Propoxyphenyl)-8-(4-phenyl-1-buten-1-yl)-chinazolin-4(3H)-on

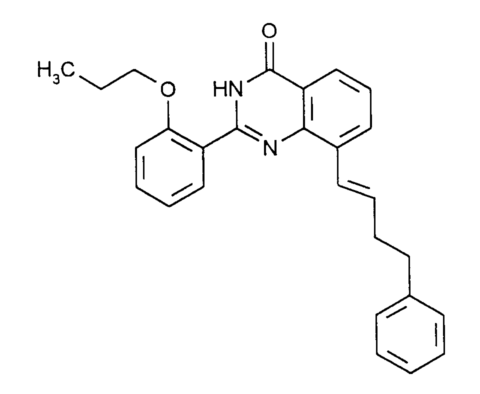

Analog der Vorschrift für Beispiel 2 wurde die Titelverbindung ausgehend von der Verbindung aus Beispiel III und 4-Phenyl-1-buten erhalten.
Ausbeute: 49,9%
R_{f} = 0,27 (Toluol/Essigsäureethylester 10:1)

### Beispiel 4 und Beispiel 5

### 2-(2-n-Propoxyphenyl)-8-(5-phenyl-2-penten-2-yl)-chinazolin-4(3H)-on und 2-(2-n-Propoxyphenyl)-8-(5-phenyl-3-penten-3-yl)-chinazolin-4(3H)-on

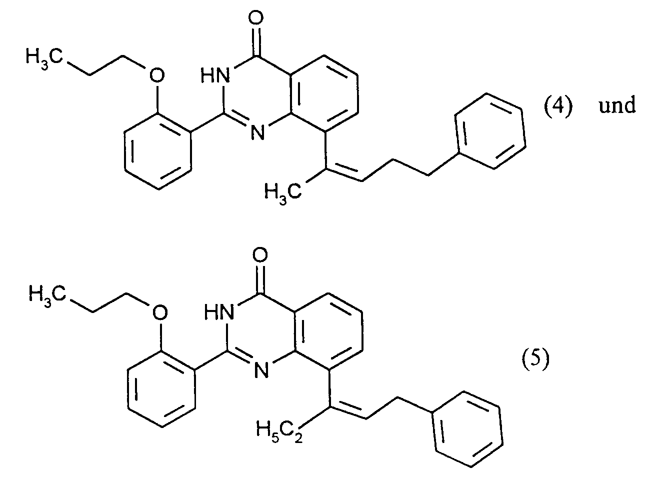

In Analogie zur Vorschrift des Beispiels 1 wurden die Titelverbindungen ausgehend von der Verbindung des Beispiels III und 5-Phenyl-penten-2 erhalten.
Ausbeute: 64,6%
Mischung der beiden Isomeren, die ohne Trennung hydriert wurden (s. Bsp. 8).

### Beispiel 6

### 2-(2-n-Propoxyphenyl)-8-(1-heptyl)-chinazolin-4(3H)-on

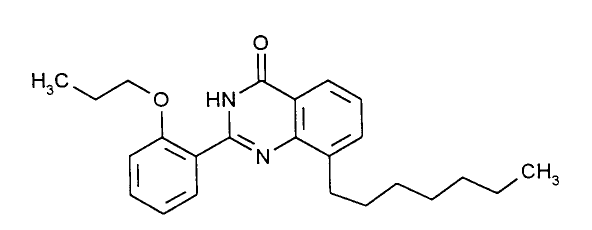

20 mg Pd/C (10%ig) wurden in 2 ml abs. Methanol 20 min. vorhydriert. Dazu wurden 200 mg (0,53 mMol) der Verbindung aus Beispiel 1 in einem Gemisch aus 2 ml abs. Methanol und 0,8 ml Essigsäureethylester gegeben und 1 h bei 20°C hydriert. Der Katalysator wurde abfiltriert, und das Lösemittel im Vakuum abrotiert. Der Rückstand war im DC sauber und kristallisierte beim Trocknen am Hochvakuum.
Ausbeute: 180 mg (89,6%)
Fp.: 73°C

### Beispiel 7

### 2-(2-n-Propoxyphenyl)-8-(3-phenyl-1-propyl)-chinazolin-4(3H)-on

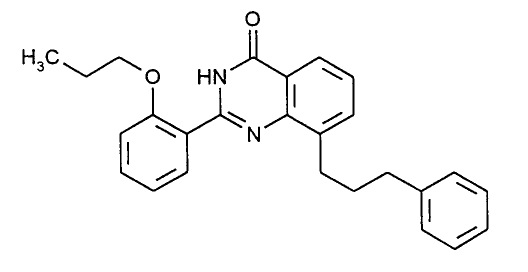

Analog der Vorschrift des Beispiels 6 wurde die Titelverbindung ausgehend von der Verbindung aus Beispiel 2 hergestellt.
Ausbeute: 79,7%
Fp.: 89°C

### Beispiel 8

### 2-(2-n-Propoxyphenyl)-8-(4-phenyl-1-butyl)-chinazolin-4(3H)-on

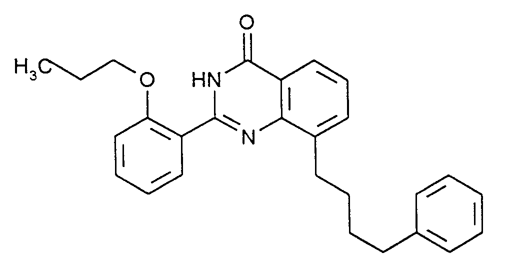

Analog der Vorschrift für Beispiel 6 wurde die Titelverbindung ausgehend von der Verbindung aus Beispiel 3 hergestellt.
Ausbeute: 86,2%
Fp.: 82°C

### Beispiel 9 und Beispiel 10

### 2-(2-n-Propoxyphenyl)-8-(5-phenyl-2-pentyl)-chinazolin-4(3H)-on und 2-(2-n-Propoxyphenyl)-8-(5-phenyl-3-pentyl)-chinazolin-4(3H)-on

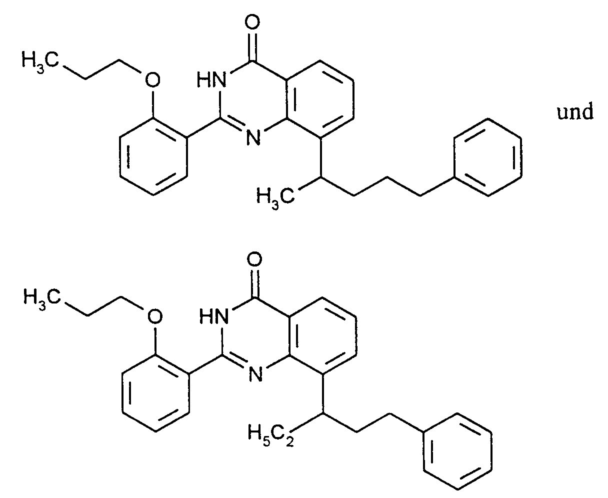

Analog der Vorschrift des Beispiels 6 wurden die Titelverbindungen ausgehend von dem Isomerengemisch aus Beispiel 4 hergestellt. Die Trennung erfolgte durch Mitteldruckchromatographie über Kieselgel mit CH₂Cl₂/Essigsäureethylester (20:5) als Eluens.
Ausbeute Bsp. 9: 9%
Ausbeute Bsp. 10:7,8%
R_{f} Bsp. 9: 0,49 (CH₂Cl₂ / Essigsäureethylester 10:1)
R_{f} Bsp. 10: 0,51 (CH₂Cl₂ / Essigsäureethylester 10:1)

### Beispiel 11

### 2-(2-n-Propoxyphenyl)-8-(1,2-epoxy-1-heptyl)-chinazolin-4(3H)-on

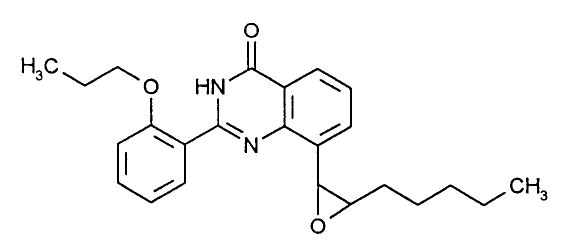

1,5 g (3,98 mMol) der Verbindung aus Beispiel 1 wurden bei 0°C in 40 ml trockenem Chloroform gelöst. Dazu wurden 0,98 g (3,98 mMol) 70%ige m-Chlorperbenzoesäure gegeben. Man ließ auf Raumtemperatur kommen und rührte 3 h nach. Es wurde 3 mal mit je 30 ml 10%iger Natriumbisulfit-Lösung und 2 mal mit je 30 ml 1 n NaOH-Lösung gewaschen, über MgSO₄ getrocknet und im Vakuum eingedampft. Der Rückstand (1,6 g) wurde über Kieselgel mit Toluol/Essigsäureethylester 95:5 als Eluens chromatographiert.
Ausbeute: 1,06 g (67,8%)
Fp.: 78°C

### Beispiel 12

### 2-(2-n-Propoxyphenyl)-8-(3-phenyl-1,2-epoxy-1-propyl)-chinazolin-4(3H)-on

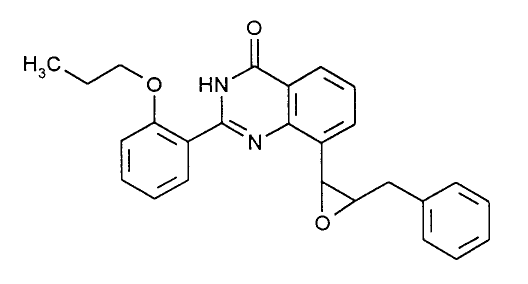

Analog der Vorschrift für Beispiel 11 wurde die Titelverbindung ausgehend von der Verbindung aus Beispiel 2 hergestellt.
Ausbeute: 47%
R_{f} =0,27 (Toluol/Essigsäureethylester 10:1)

### Beispiel 13

### 2-(2-n-Propoxyphenyl)-8-(4-phenyl-1,2-epoxy-1-butyl)-chinazolin-4(3H)-on

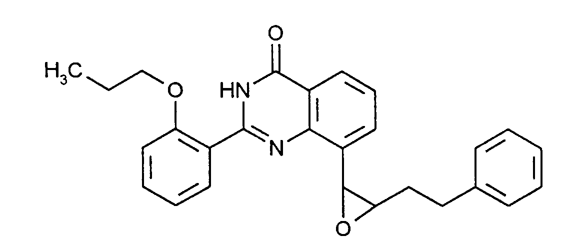

Analog der Vorschrift für Beispiel 11 wurde die Titelverbindung ausgehend von der Verbindung aus Beispiel 3 hergestellt.
Ausbeute: 61,4%
R_{f} = 0,29 (Toluol/Essigsäureethylester 1:1)

### Beispiel 14

### 2-(2-n-Propoxyphenyl)-8-(1-methoxy-2-hydroxy-1-heptyl)-chinazolin-4(3H)-on

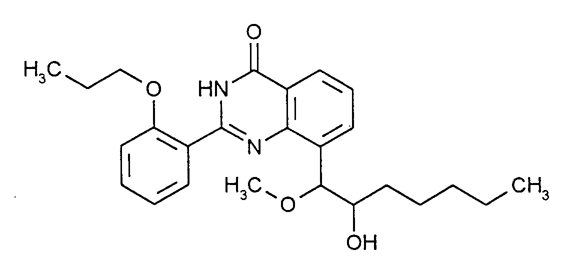

Zu einer Lösung von 0,2 g (0,51 mMol) der Verbindung aus Beispiel 11 in 6 ml Methanol wurden bei 0°C 0,1 ml Bortrifluorid-etherat (0,76 mMol) getropft. Nach 20 min. bei 0°C wurden 75 ml Essigsäureethylester zugegeben und 3 mal mit je 50 ml Wasser ausgeschüttelt. Die organische Phase wurde über Kieselgel mit Toluol/Essigsäureethylester 5:1 als Eluens chromatographiert.
Ausbeute: 160 mg (73,9%)
R_{f} 0,19 (Toluol/Essigsäureethylester 5:1)

### Beispiel 15

### 2-(2-n-Propoxyphenyl)-8-(3-phenyl-1-methoxy-2-hydroxy-1-propyl)-chinazolin-4(3H)-on

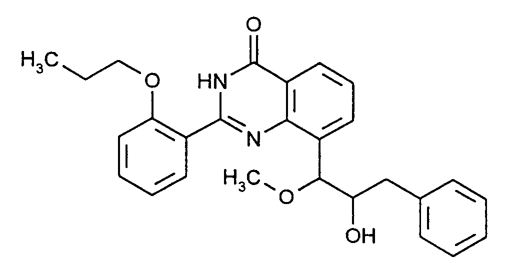

Analog der Vorschrift für Beispiel 14 wurde die Titelverbindung ausgehend von der Verbindung aus Beispiel 12 hergestellt.
Ausbeute: 32,5%
R_{f} = 0,20 (Toluol/Essigsäureethylester 5:1)

### Beispiel 16

### 2-(2-n-Propoxyphenyl)-8-(4-phenyl-1-methoxy-2-hydroxy-1-butyl)-chinazolin-4(3H)-on

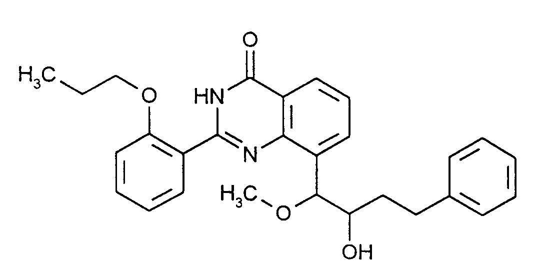

Analog der Vorschrift für Beispiel 14 wurde die Titelverbindung ausgehend von der Verbindung aus Beispiel 10 hergestellt.
Ausbeute: 74,4%
R_{f} = 0,17 (Toluol/Essigsäureethylester 5:1)

### Beispiel 17

### 2-(2-n-Propoxyphenyl)-8-(3-hydroxy-2-octyl)-chinazolin-4(3H)-on

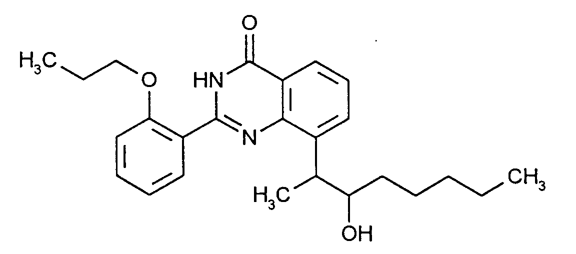

Zu einer Suspension von 0,14 g (1,53 mMol) Cu(I)CN in 3 ml abs Diethylether wurden bei -78°C 1,9 ml 1,6 molare Methyllithiumlösung in Diethylether (3,06 mMol) zugetropft. Nach 1 h bei -78°C wurde auf -45°C erwärmt und 200 mg (0,51 mMol) der Verbindung aus Beispiel 11 in 2 ml abs. Diethylether zugetropft. Man rührte 1 h bei 0°C, dann bei 20°C bis die Reaktion beendet (DC-Kontrolle, ca. 1 h) war. Nach Zugabe von 50 ml Essigsäureethylester wurde 3 mal mit je 30 ml Wasser gewaschen. Die organische Phase wurde über Na₂SO₄ getrocknet und im Vakuum einrotiert. Der Rückstand wurde über Kieselgel mit Toluol/Essigsäureethylester 7:1 als Eluens chromatographiert.
Ausbeute: 80 mg (38,4%)
R_{f} = 0,22 (Toluol/Essigsäureethylester 5:1)

### Beispiel 18

### 2-(2-n-Propoxyphenyl)-8-(4-phenyl-3-hydroxy-2-butyl)-chinazolin-4(3H)-on

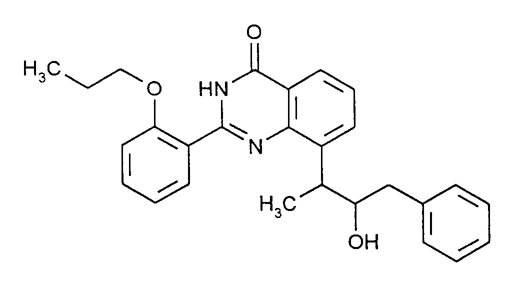

Analog der Vorschrift für Beispiel 14 wurde die Titelverbindung ausgehend von der Verbindung des Beispiels 9 hergestellt.
Ausbeute: 38,5%
R_{f} = 0,21 (Toluol/Essigsäureethylester 5:1)

### Beispiel 19

### 2-(2-n-Propoxyphenyl)-8-(5-phenyl-3-hydroxy-2-pentyl)-chinazolin-4(3H)-on

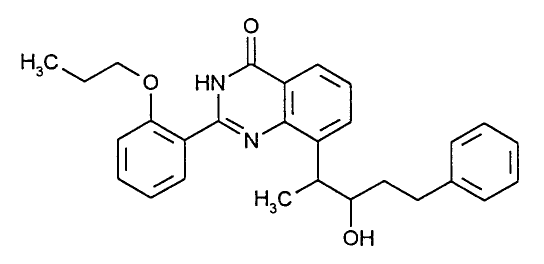

Analog der Vorschrift für Beispiel 17 wurde die Titelverbindung ausgehend von der Verbindung aus Beispiel 13 hergestellt.
Ausbeute: 51,4%
Diasteromerengemisch R_{f} = 0,18 und 0,24 (Toluol/Essigsäureethylester 5:1)

### Beispiel 20

### 2-(2-n-Propoxyphenyl)-8-(4-hydroxy-3-nonyl)-chinazolin-4(3H)-on

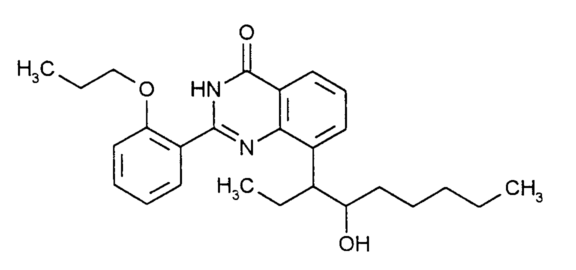

1,02 ml einer 3 M C₂H₅MgBr-Lösung (3,05 mMol) in Diethylether wurde bei -20°C zu einer Lösung von 240 mg (0,61 mMol) der Verbindung aus Beispiel 11 getropft und 45 min bei -20°C, dann 20 min bei Raumtemperatur gerührt. Durch Zugabe von 4 ml abs. Tetrahydrofuran wurde der ölige Niederschlag aufgelöst und noch einmal 1,02 ml der 3 M C₂H₅MgBr-Lösung zur Vervollständigung der Umsetzung zugegeben. Nach 15 min bei 20°C wurden 75 ml Essigsäureethylester zugegeben und 3 mal mit je 50 ml Wasser ausgeschüttelt. Nach Trocknen der organischen Phase über MgSO₄ wurde das Lösemittel im Vakuum einrotiert, und der Rückstand über Kieselgel mit Toluol/Essigsäureethylester 10:1 als Eluens chromatographiert.
Ausbeute: 40 mg (15,5%)
R_{f} = 0,24 (Toluol/Essigsäurethylester 5:1)

### Beispiel 21

### 2-(2-n-Propoxyphenyl)-8-(3-methansulfonyloxy-2-octyl)-chinazolin-4(3H)-on

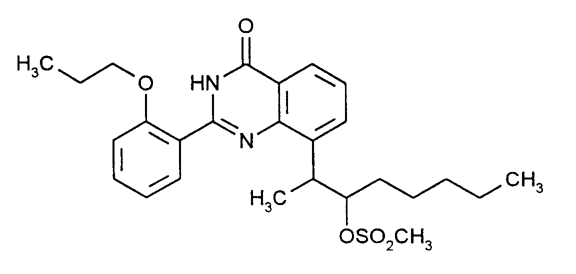

740 mg (1,81 mMol) der Verbindung aus Beispiel 17 und 0,3 ml (2,17 mMol) Triethylamin in 18 ml abs. CH₂Cl₂ wurden bei 0°C mit 0,17 ml (2,17 mMol) Methansulfonsäurechlorid versetzt. Man ließ auf Raumtemperatur kommen und rührte 30 min nach. Man schüttelte 2 mal mit je 30ml 1 n NaOH und 2 mal mit je 30 ml 1 n HCl aus, trocknete die organische Phase über MgSO₄ und rotierte das Lösemittel im Vakuum ab. Der feste Rückstand wurde in einem Gemisch aus 30 ml Essigsäureethylester und 30 ml Petrolether verrührt und das Produkt abfiltriert. Ausbeute: 650 mg (73,8%)
Fp.: 195°C

### Beispiel 22

### 2-(2-n-Propoxyphenyl)-8-(3-azido-2-octyl)-chinazolin-4(3H)-on

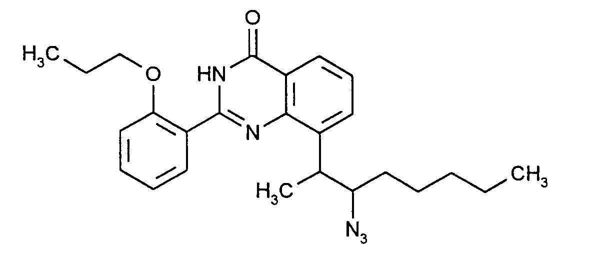

50 mg (0,103 mMol) der Verbindung aus Beispiel 18 und 13,4 ml (0,206 mMol) Natriumazid wurden in 2 ml abs. DMF über Nacht bei 40°C gerührt. Es wurden 5 ml Essigsäureethylester zugegeben und 3 mal mit je 50 ml Wasser ausgeschüttelt. Nach Trocknen der organischen Phase über Na₂SO₄ wurde das Lösemittel im Vakuum abrotiert, und der Rückstand durch Flashchromatographie über Kieselgel (Eluens: Toluol/Essigsäureethylester 5:1) gereinigt.
Ausbeute: 31 mg (67%)
R_{f} = 0,59 (Toluol/Essigsäureethylester 5:1)

### Beispiel 23

### 2-(2-n-Propoxyphenyl)-8-(1-methoxy-2-oxo-1-heptyl)-chinazolin-4(3H)-on

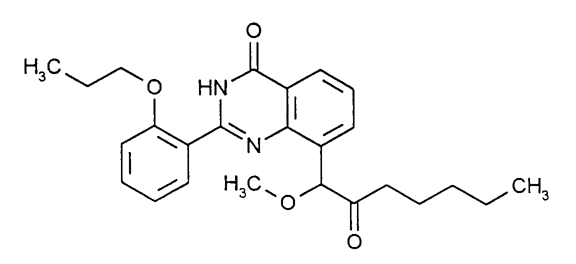

Zu 0,21 ml (2,46 mMol) Oxalylchlorid in 13 ml abs. CH₂Cl₂ wurde bei -70°C 0,38 ml (5,41mMol) abs. DMSO in 4 ml abs. CH₂Cl₂ zugetropft. Nach 30 min. wurden 870 mg (2,05mMol) der Verbindung aus Beispiel 14 in 6 ml abs. CH₂Cl₂, nach weiteren 30 min. 1,42 ml (10,24 mMol) N(C₂H₅)₃ zugetropft. Man ließ auf RT kommen und versetzte nach 10 min. mit 100 ml H₂O. Die wäßrige Phase wurde 3 mal mit je 50 ml CH₂Cl₂ extrahiert, die vereinigten CH₂Cl₂-Phasen über MgSO₄ getrocknet und einrotiert. Der Rückstand wurde in 10 ml Ethanol gelöst und nach Zugabe von 3 ml 1 n HCl 3 h bei RT gerührt. Das Ethanol wurde im Vakuum abgedampft, der Rückstand in 30 ml Essigsäureethylester aufgenommen und 2 mal mit H₂O gewaschen. Nach Trocknen über MgSO₄ wurde im Vakuum einrotiert und der Rückstand durch Chromatographie über Kieselgel mit Toluol / Essigsäureethylester 98:2 als Eluens gereinigt.
Ausbeute: 510 mg (58,9%)
R_{f} = 0,26 (Toluol / Essigsäureethylester 5:1)

### Beispiel 24

### 2-(2-n-Propoxy-5-morpholinosulfonylphenyl)-8-(1-hepten-1-yl)-chinazolin-4(3H)-on

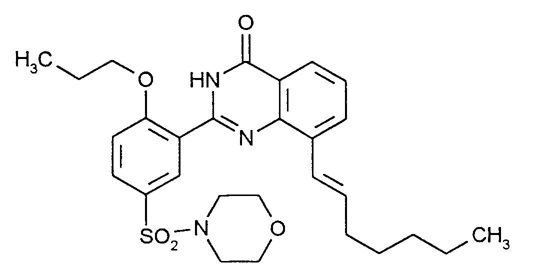

In Analogie zur Voschrift des Beispiels 1 wurde die Titelverbindung ausgehend von 2-(2-n-Propoxy-5-morpholinosulfonylphenyl)-8-brom-chinazolin-4-(3H)-on und 1-Hepten erhalten.
Ausbeute: 53,2%
Fp.: 112°C (Diethylether)

### Beispiel 25

### 2-(2-n-Propoxy-5-morpholinosulfonylphenyl)-8-(1,2-epoxy-1-heptyl)-chinazolin-4-(3H)-on

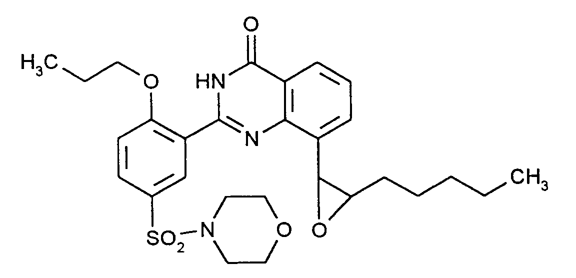

In Analogie zur Vorschrift des Beispiels 11 wurde die Titelverbindung ausgehend von der Verbindung aus Beispiel 24 hergestellt.
Ausbeute: 90.7%
Fp.: 96°C

### Beispiel 26

### 2-(2-n-Propoxy-5-morpholinosulfonylphenyl)-8-(1-methoxy-2-hydroxy-1-heptyl)chinazolin-4-(3H)-on

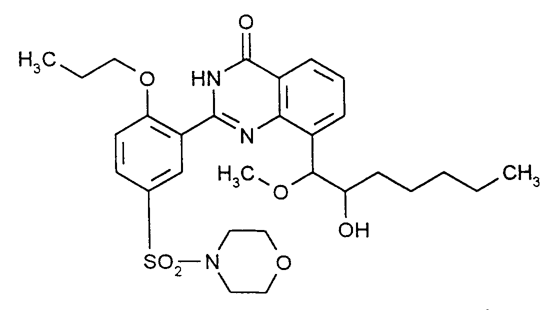

In Analogie zur Vorschrift des Beispiels 14 wurde die Titelverbindung ausgehend von der Verbindung aus Beispiel 25 hergestellt.
Ausbeute: 20,3%
R_{f} = 0,42 (Toluol / Essigsäureethylester 2.1)

### Beispiel 27 und Beispiel 28

### 2-(2-n-Propoxy-5-morpholinosulfonylphenyl)-8-(5-phenyl-2-penten-2-yl)-chinazolin-4-(3H)-on und 2-(2-n-Propoxy-5-morpholinosulfonylphenyl)-8-(5-phenyl-3-penten-3-yl)-chinazolin-4-(3H)-on

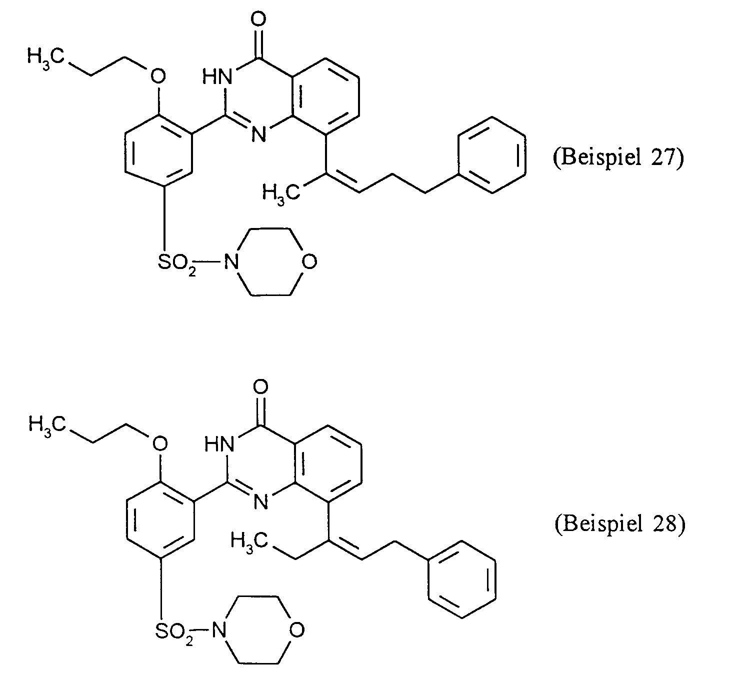

In Analogie zur Vorschrift des Beispiels 1 wurden die Titelverbindungen ausgehend von 2-(2-n-Propoxy-5-morpholinosulfonylphenyl)-8-brom-chinazolin-4-(3H)-on und 5-Phenyl-2-penten erhalten.
Ausbeute: 39%
Mischung der beiden Isomeren, die ohne Trennung hydriert wurden.

### Beispiel 29 und Beispiel 30

### 2-(2-n-Propoxy-5-morpholinosulfonylphenyl)-8-(5-phenyl-2-pentyl)-chinazolin-4-(3H)-on und 2-(2-n-Propoxy-5-morpholinosulfonylphenyl)-8-(5-phenyl-3-pentyl)chinazolin-4-(3H)-on

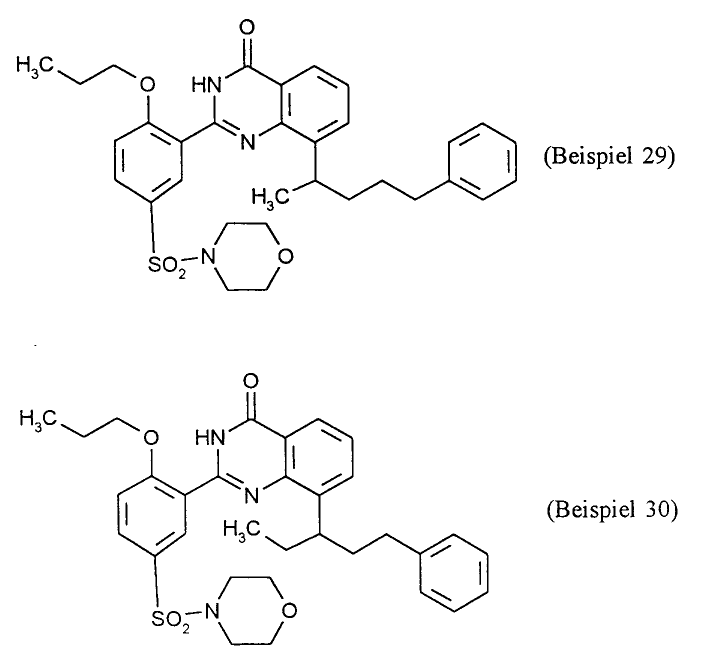

In Analogie zur Vorschrift des Beispiels 6 wurden die Titelverbindungen ausgehend von den Isomerengemischen aus Beispiel 27 hergestellt. Die Trennung erfolgte durch Mitteldruckchromatographie über Kieselgel mit CH₂Cl₂/Essigsäureethylester (2:1) als Eluens.
Ausbeute Bsp. 29: 36,3% R_{f} = 0,44 (CH₂Cl₂ / Essigsäureethylester 4:1)
Ausbeute: Bsp. 30: 18,4% R_{f} = 0,49 (CH₂Cl₂ / Essigsäureethylester 4:1)

## Patentansprüche

1. 2,8-Disubstituierte Chinazolinone der allgemeinen Formel (I) 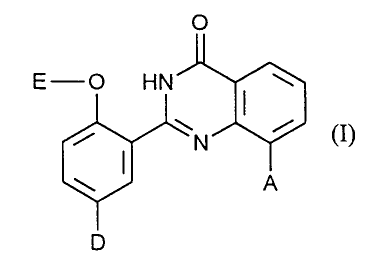 in welcher
A für Oxiranyl steht, das gegebenenfalls durch geradkettiges oder verzweigtes Alkyl mit bis zu 8 Kohlenstoffatomen substituiert ist, das seinerseits durch Phenyl substituiert sein kann, oder für einen Rest der Formel 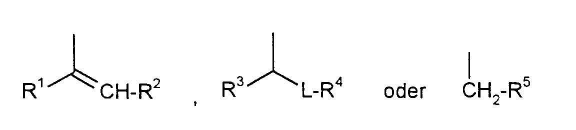 steht,
worin
R¹ Wasserstoff oder geradkettiges oder verzweigtes Alkyl mit bis zu 6 Kohlenstoffatomen bedeutet,
R² geradkettiges oder verzweigtes Alkyl mit bis zu 8 Kohlenstoffatomen bedeutet, das gegebenenfalls durch Phenyl substituiert ist,
R³ geradkettiges oder verzweigtes Alkyl mit bis zu 5 Kohlenstoffatomen oder eine Gruppe der Formel -OR⁶ bedeutet,
worin
R⁶ Wasserstoff, eine Hydroxyschutzgruppe oder geradkettiges oder verzweigtes Alkyl mit bis zu 5 Kohlenstoffatomen bedeutet,
R⁴ geradkettiges oder verzweigtes Alkyl mit 2 bis 10 Kohlenstoffatomen bedeutet, das gegebenenfalls durch Phenyl substituiert ist,
L einen Rest der Formel -CO-, -CH(OH), -CH₂, -CH(N₃) oder -CH(OSO₂R⁷) bedeutet,
worin
R⁷ geradkettiges oder verzweigtes Alkyl mit bis zu 4 Kohlenstoffatomen oder Phenyl bedeutet,
R⁵ geradkettiges oder verzweigtes Alkyl mit 3 bis 8 Kohlenstoffatomen bedeutet, das gegebenenfalls durch Phenyl substituiert ist, oder
Benzyl oder 2-Phenylethyl bedeutet,
D für Wasserstoff oder für eine Gruppe der Formel -SO₂-NR⁸R⁹ steht,
worin
R⁸ und R⁹ gleich oder verschieden sind und
Wasserstoff, Phenyl oder geradkettiges oder verzweigtes Alkyl mit bis zu 6 Kohlenstoffatomen bedeuten, das gegebenenfalls durch Hydroxy substituiert ist,
oder gemeinsam mit dem Stickstoffatom einen 5- bis 6-gliedrigen gesättigten Heterocyclus mit bis zu 2 weiteren Heteroatomen aus der Reihe S, N und/oder O bilden, der gegebenenfalls, auch über eine freie N-Funktion, durch geradkettiges oder verzweigtes Alkyl mit bis zu 6 Kohlenstoffatomen substituiert ist, das seinerseits durch Hydroxy substituiert sein kann,
E für geradkettiges oder verzweigtes Alkyl mit bis zu 8 Kohlenstoffatomen steht
und deren Tautomere und Salze.

2. 2,8-Disubstituierte Chinazolinone der Formel nach Anspruch 1
in welcher
A für Oxiranyl steht, das gegebenenfalls durch geradkettiges oder verzweigtes Alkyl mit bis zu 7 Kohlenstoffatomen substituiert ist, das seinerseits durch Phenyl substituiert sein kann, oder für einen Rest der Formel 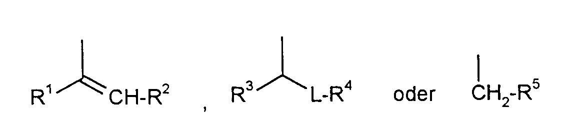 steht,
worin
R¹ Wasserstoff oder geradkettiges oder verzweigtes Alkyl mit bis zu 5 Kohlenstoffatomen bedeutet,
R² geradkettiges oder verzweigtes Alkyl mit bis zu 6 Kohlenstoffatomen bedeutet, das gegebenenfalls durch Phenyl substituiert ist,
R³ geradkettiges oder verzweigtes Alkyl mit bis zu 4 Kohlenstoffatomen oder eine Gruppe der Formel -OR⁶ bedeutet,
worin
R⁶ Wasserstoff, Benzyl, Acetyl oder geradkettiges oder verzweigtes Alkyl mit bis zu 4 Kohlenstoffatomen bedeutet,
R⁴ geradkettiges oder verzweigtes Alkyl mit 2 bis 8 Kohlenstoffatomen bedeutet, das gegebenenfalls durch Phenyl substituiert ist,
L einen Rest der Formel -CO-, -CH(OH), -CH₂, -CH(N₃) oder -CM(OSO₂R⁷) bedeutet,
worin
R⁷ geradkettiges oder verzweigtes Alkyl mit bis zu 3 Kohlenstoffatomen oder Phenyl bedeutet,
R⁵ geradkettiges oder verzweigtes Alkyl mit 3 bis 7 Kohlenstoffatomen bedeutet, das gegebenenfalls durch Phenyl substituiert ist, oder
Benzyl oder 2-Phenylethyl bedeutet,
D für Wasserstoff oder für eine Gruppe der Formel -SO₂-NR⁸R⁹ steht,
worin
R⁸ und R⁹ gleich oder verschieden sind und
Wasserstoff, Phenyl oder geradkettiges oder verzweigtes Alkyl mit bis zu 5 Kohlenstoffatomen bedeuten, das gegebenenfalls durch Hydroxy substituiert ist,
oder gemeinsam mit dem Stickstoffatom einen Morpholinyl-, Piperidinyl- oder Piperazinylring bilden, der gegebenenfalls, auch über eine freie N-Funktion, durch geradkettiges oder verzweigtes Alkyl mit bis zu 4 Kohlenstoffatomen substituiert ist, das seinerseits durch Hydroxy substituiert sein kann,
E für geradkettiges oder verzweigtes Alkyl mit bis zu 6 Kohlenstoffatomen steht
und deren Tautomere und Salze.

3. 2,8-disubstituierte Chinazolinone der Formel nach Anspruch 1
in welcher
A für Oxiranyl steht, das gegebenenfalls durch geradkettiges oder verzweigtes Alkyl mit bis zu 6 Kohlenstoffatomen substituiert ist, das seinerseits durch Phenyl substituiert sein kann, oder für einen Rest der Formel 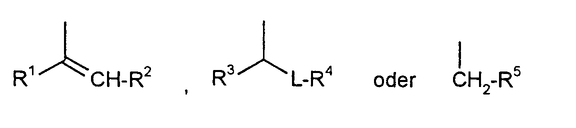 steht,
worin
R¹ Wasserstoff oder geradkettiges oder verzweigtes Alkyl mit bis zu 3 Kohlenstoffatomen bedeutet,
R² geradkettiges oder verzweigtes Alkyl mit bis zu 6 Kohlenstoffatomen bedeutet, das gegebenenfalls durch Phenyl substituiert ist,
R³ geradkettiges oder verzweigtes Alkyl mit bis zu 4 Kohlenstoffatomen oder eine Gruppe der Formel -OR⁶ bedeutet,
worin
R⁶ Wasserstoff Benzyl, Acetyl oder geradkettiges oder verzweigtes Alkyl mit bis zu 3 Kohlenstoffatomen bedeutet,
R⁴ geradkettiges oder verzweigtes Alkyl mit 2 bis 7 Kohlenstoffatomen bedeutet, das gegebenenfalls durch Phenyl substituiert ist,
L einen Rest der Formel -CO-, -CH(OH), -CH₂, -CH(N₃) oder -CH(OSO₂R⁷) bedeutet,
worin
R⁷ geradkettiges oder verzweigtes Alkyl mit bis zu 3 Kohlenstoffatomen oder Phenyl bedeutet,
R⁵ geradkettiges oder verzweigtes Alkyl mit 3 bis 6 Kohlenstoffatomen bedeutet, das gegebenenfalls durch Phenyl substituiert ist, oder
Benzyl oder 2-Phenylethyl bedeutet,
D für Wasserstoff oder für eine Gruppe der Formel -SO₂-NR⁸R⁹ steht,
worin
R⁸ und R⁹ gleich oder verschieden sind und
Wasserstoff, Phenyl oder geradkettiges oder verzweigtes Alkyl mit bis zu 3 Kohlenstoffatomen bedeuten,
oder gemeinsam mit dem Stickstoffatom einen Morpholinyl- oder Piperidinylring bilden,
E für geradkettiges oder verzweigtes Alkyl mit bis zu 4 Kohlenstoffatomen steht
und deren Tautomere und Salze.

4. 2,8-Disubstituierte Chinazolinone nach Anspruch 1 bis 3 zur therapeutischen Anwendung.

5. Verfahren zur Herstellung von 2,8-disubstituierten Chinazolinonen nach Anspruch 1 bis 3, dadurch gekennzeichnet, daß man zunächst Verbindungen der allgemeinen Formel (II) 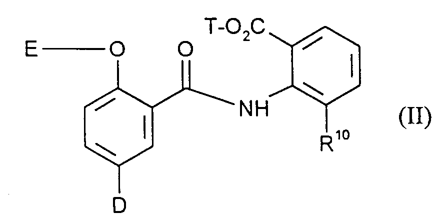 in welcher
D und E die oben angegebene Bedeutung haben,
T für C₁-C₄-Alkyl steht
und
R¹⁰ für Halogen, vorzugsweise für Brom oder Joed steht mit Formamid zu den Verbindungen der allgemeinen Formel (III) 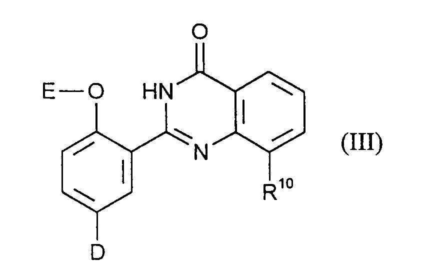 in welcher
D, E und R¹⁰ die oben angegebene Bedeutung haben,
cyclisiert,
und in einem letzten Schritt mit Verbindungen der allgemeinen Formel (IV)
R¹-CH=CH-R² (IV)
in welcher
R¹ und R² die oben angegebene Bedeutung haben,
in inerten Lösemitteln, in Anwesenheit einer Base und im System Tri-o-tolylphosphin/Palladium-II-acetat in die Verbindungen der allgemeinen Formel (Ia) 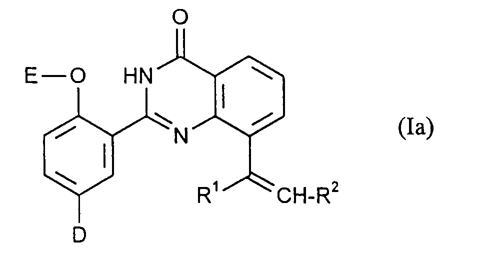 in welcher
D, E, R¹ und R² die oben angegebene Bedeutung haben,
überführt,
und gegebenenfalls die Doppelbindung hydriert,
oder im Fall A = substituiertes Oxiranyl,
gegebenenfalls die Doppelbindung nach üblichen Methoden mit einem Oxidationsmittel in inerten Lösemitteln zu den entsprechenden Epoxyverbindungen oxidiert und diese durch Ringöffnungsreaktionen in die entsprechenden Hydroxyverbindungen überführt,
und ausgehend von den Hydroxyverbindungen, gegebenenfalls nach Aktivierung, nucleophile Substitutionsreaktionen durchführt,
oder die Hydroxyverbindungen zu den Oxoverbindungen oxidiert.

6. Arzneimittel enthaltend mindestens ein 2,8-disubstituiertes Chinazolinon nach Anspruch 1 bis 3.

7. Arzneimittel nach Anspruch 6, zur Behandlung von Entzündungen thromboembolischen Erkrankungen sowie Herz- und Kreislauferkrankungen.

8. Arzneimittel nach Anspruch 6 und 7 zur Behandlung von Impotenz.

9. Verwendung von 2,8-disubstituierten Chinazolinonen nach Anspruch 1 bis 3, zur Herstellung von Arzneimitteln.

10. Verwendung nach Anspruch 9, dadurch gekennzeichnet, daß die Arzneimittel zur Behandlung von Entzündungen, thromboembolischen sowie Herz- und Kreislauferkrankungen eingesetzt werden.
